# NOUVEAU FASCICULE DE BREVET EUROPEEN

(11) **EP 2 189 135 B3**
(45) Date de publication du présent fascicule: **08.06.2016**
(45) Mention de la délivrance du brevet: 23.11.2011
(21) Numéro de dépôt: 10153901.3
(22) Date de dépôt: 08.01.2008
(51) Int. Cl.: A61F 2/34, A61F 2/46

(54) **Cotyle a interface sterile**
Gelenkpfanne mit steriler Fläche
Acetabular cup comprising a sterile interface

(30) Priorité: 08.01.2007 FR 0752558
(43) Date de publication de la demande: 26.05.2010
(62) Demande divisionnaire de: 08700220.0
(73) Titulaire: Gradel, Thomas, 74970 Marignier (FR)
(72) Inventeur: Gradel, Thomas, 74970 Marignier (FR)
(74) Mandataire: Poncet, Jean-François

(56) Documents cités:
- DE-A1- 19 640 747
- US-A1- 2006 241 781
- US-B1- 6 682 567

## Description

### DOMAINE TECHNIQUE DE L'INVENTION

La présente invention concerne un cotyle prothétique destiné à remplacer le cotyle naturel de la hanche.

Une prothèse totale de hanche comprend deux parties constituant une articulation à rotule, à savoir une partie femelle destinée à remplacer le cotyle naturel de la hanche et une partie mâle destinée à remplacer la tête du fémur.

La partie mâle de l'articulation comporte généralement une tige, destinée à pénétrer dans le canal médullaire du fémur, et dont l'extrémité proximale se raccorde par un col à une tête sphérique destinée à pénétrer dans le cotyle.

La partie femelle de l'articulation, qui doit remplacer le cotyle naturel de la hanche, et que l'on désignera globalement par l'appellation cotyle, comprend habituellement une cupule d'insertion hémisphérique, qui vient se loger dans une cavité cotyloïdienne préparée de l'os du bassin, et dans laquelle on loge un insert articulaire définitif. De façon courante, la cupule d'insertion est métallique.

L'insert articulaire est en une matière à faible coefficient de frottement tel que le polyéthylène ou une céramique.

Dans les cotyles à simple mobilité, l'insert en polyéthylène ou en céramique est fixe dans la cupule d'insertion et comporte une cavité articulaire sensiblement hémisphérique coaxiale qui permet l'engagement et le pivotement de la tête sphérique de la partie mâle de l'articulation. Les mouvements de rotation de l'articulation se font alors entre la tête sphérique de la partie mâle de prothèse et la cavité articulaire de l'insert.

Lors de la pose de la cupule d'insertion dans la cavité cotyloïdienne, il faut pouvoir utiliser un impacteur qui permet de tenir et manipuler la cupule d'insertion et de lui appliquer une force d'enfoncement dans la cavité cotyloïdienne de l'os avec une bonne orientation, pendant une durée suffisante notamment pour la prise d'un ciment entre la surface externe de la cupule d'insertion et la cavité cotyloïdienne de l'os.

Dans les cotyles à simple mobilité, l'impacteur comporte généralement un tronçon d'extrémité fileté qui vient se fixer dans un trou fileté prévu au centre de la cupule d'insertion.

Le principal problème lors de l'utilisation d'une prothèse de hanche est le risque de luxation. La luxation est une sortie de la tête fémorale sphérique hors de la cavité articulaire.

Pour réduire les risques de luxation, on a proposé des structures de cotyles à double mobilité, dans lesquels la cupule d'insertion est métallique et reçoit un insert articulaire en polyéthylène qui est lui-même monté rotatif dans la cupule d'insertion. Un inconvénient de cette structure est l'apparition d'une usure progressive de l'insert articulaire, qui engendre, après quelques années d'utilisation, une instabilité de l'articulation et un risque de luxation.

Un autre moyen efficace pourempêcher la luxation est d'utiliser, dans un cotyle à simple mobilité, une tête fémorale prothétique sphérique de grand diamètre. Pour faire échapper la tête fémorale sphérique de la cavité articulaire, il est en effet nécessaire de faire sortir la tête fémorale d'une distance sensiblement égale au rayon de la tête fémorale. Ainsi, plus le diamètre de la tête fémorale prothétique est grand, plus l'effort nécessaire pour luxer la prothèse de hanche doit être important.

La cavité cotyloïdienne d'un patient a cependant des dimensions fixes qui peuvent très difficilement (voire pas du tout) être modifiées. L'épaisseur nécessaire de l'insert articulaire immobile détermine alors le diamètre possible de la tête fémorale prothétique. On a donc imaginé d'utiliser un insert articulaire immobile de faible épaisseur pour permettre une augmentation du diamètre de la tête fémorale prothétique. Pour réduire encore l'épaisseur de l'insert articulaire immobile au profit du diamètre de la tête fémorale, tout en réduisant les risques d'usure, on a imaginé des inserts à cupule d'insertion métallique et insert en céramique, la céramique présentant à la fois de bonnes propriétés de glissement et une résistance mécanique plus élevée que le polyéthylène. La résistance mécanique plus élevée de la céramique lui permet de supporter les contraintes mécaniques de l'articulation tout en ayant une épaisseur réduite. Avec les céramiques actuelles, on peut utiliser des inserts articulaires immobiles dont l'épaisseur réduite est inférieure ou égale à 4 mm environ. Un tel cotyle à insert en céramique est décrit dans le document EP 1 290 992 A1.

Pour encore augmenter le diamètre de la tête fémorale prothétique, ce document enseigne de réduire l'épaisseur de la cupule d'insertion, en conservant une même épaisseur de l'insert immobile en céramique. Ainsi, la cupule métallique a une épaisseur comprise entre 0,1 et 2 mm, et l'insert en céramique a une épaisseur inférieure à 4 mm.

Mais on constate alors une augmentation importante des risques de rupture de l'insert en céramique, soit lors de l'impaction du cotyle poursa pose, soit même lors de l'utilisation ultérieure de la prothèse.

Selon la présente invention, on considère que ces ruptures proviennent d'une répartition inégale des efforts mécaniques sur l'insert en céramique, étant observé que le document EP 1 290 992 A1 ne mentionne pas ces difficultés et ne décrit aucun moyen permettant l'impaction correcte du cotyle.

### EXPOSE DE L'INVENTION

Le problème proposé par la présente invention est à la fois de diminuer le risque de luxation d'une prothèse de hanche et de réduire les risques de rupture de l'insert en céramique tant lors de son impaction dans la cavité cotyloïdienne du bassin d'un patient que lors de l'utilisation ultérieure de la prothèse.

Pour atteindre ces buts ainsi que d'autres, l'invention propose un procédé de fabrication de cotyle prothétique de hache comme défini dans la revendication 1.

Grâce à la faible épaisseur de la cupule d'insertion et de l'insert articulaire, une dimension maximale peut être donnée à la tête fémorale et à la surface articulaire concave pour réduire efficacement les risques de luxation de l'articulation lors de l'usage ultérieur. Simultanément, malgré ces épaisseurs réduites, la structure annulaire de réception permet non seulement une tenue efficace du cotyle lors de son impaction par un impacteur, mais aussi une coopération efficace entre la cupule d'insertion et l'insert articulaire qui sont assemblés avant l'impaction. Cette coopération réduit les risques de déformation de la cupule d'insertion pendant l'impaction car l'insert articulaire en céramique reste au contact de la cupule d'insertion et supporte une partie des efforts. Et cette coopération réduit les risques de rupture de l'insert articulaire pendant l'impaction, les efforts d'impaction étant appliqués sur la cupule d'insertion qui les transmet ensuite de façon répartie sur l'insert articulaire en céramique. Après l'impaction, cette coopération réduit aussi les risques de rupture ultérieure de l'insert articulaire, par le fait que les efforts mécaniques sont transmis de façon régulière et répartie entre l'insert articulaire et la cupule d'insertion qui n'a pas été déformée.

Lors de la pose, la cupule d'insertion reste solidaire de l'insert articulaire immobile qui est définitif. L'interface entre ces deux pièces reste donc inaccessible et stérile, sans risque de contamination pendant l'opération.

Grâce au fait que la structure périphérique de réception est en saillie au-delà de la face réceptrice de l'insert articulaire immobile définitif inséré dans la cupule d'insertion, et grâce à l'épaisseur suffisante de la cupule d'insertion au voisinage de son bord annulaire, c'est-à-dire dans la zone constituant la structure périphérique de réception, une fixation fiable de l'impacteur à la cupule d'insertion est possible, malgré la présence de l'insert articulaire immobile définitif qui empêche tout accès à un quelconque trou fileté prévu au centre de la cupule d'insertion.

En outre, dans les positions extrêmes d'utilisation de la prothèse, le col prothétique portant la tête fémorale prothétique vient en appui contre la structure périphérique de réception en saillie. Le col prothétique ne vient ainsi pas buter contre l'insert en céramique immobile définitif et ne risque donc pas d'endommager celui-ci par l'application d'un effort localisé ponctuel.

De préférence, la face annulaire de réception se développe en continuité de la face de réception concave de la cupule d'insertion et est ainsi une portion annulaire de la face intérieure de la cupule d'insertion. Une telle structure périphérique de réception est peu encombrante et facile à réaliser par des moyens courants de fabrication. Cette structure périphérique de réception est également d'un seul tenant avec la cupule d'insertion et est donc fiable pour permettre au chirurgien d'appliquer les efforts nécessaires pour une bonne fixation et une bonne orientation de la prothèse.

Le cotyle est associé à un insert de pose et d'orientation, fixé de façon amovible à la structure périphérique de réception de la cupule d'insertion, et comportant une structure d'assemblage sur laquelle l'impacteur peut être fixé de façon amovible. Un tel insert, fixé selon la périphérie de la cupule d'insertion, permet de bien répartir sur la cupule les efforts appliqués par le chirurgien pour fixer et orienter la prothèse.

Selon l'invention, le cotyle est conditionné assemblé à l'état stérile, avec l'insert articulaire immobile définitif en céramique engagé dans la cupule d'insertion, et avec l'insert de pose et d'orientation fixé à la cupule d'insertion.

Un intérêt d'une telle combinaison est que l'insert de pose et d'orientation assure le maintien de l'insert articulaire en céramique dans la cupule d'insertion pendant le transport et les manipulations. On peut ainsi prévoir un insert articulaire qui est simplement engagé dans la cupule d'insertion, et qui peut ensuite en être extrait si le chirurgien le souhaite, sans pour autant qu'il y ait un risque d'extraction intempestive avant la pose du cotyle. Cette combinaison peut être utilisée indépendamment des caractéristiques d'épaisseur de l'insert articulaire et de la cupule d'insertion.

Pour améliorer encore la tenue de l'insert articulaire, on peut prévoir que l'insert de pose et d'orientation fixé sur la face annulaire de réception de la cupule d'insertion est en appui sur ou à faible écart de la face intérieure réceptrice de l'insert articulaire immobile définitif en céramique.

En alternative ou en complément, on peut prévoir que l'insert de pose et d'orientation, fixé sur la face annulaire de réception, comporte une ou plusieurs nervures élastiques en saillie venant en appui sur l'insert articulaire immobile définitif en céramique pour le maintenir dans la cupule d'insertion.

L'insert de pose et d'orientation peut être en polyéthylène, matière peu onéreuse et aisément stérilisable qui peut être utilisée dans le milieu chirurgical.

De préférence, la structure d'assemblage peut comprendre un trou de fixation taraudé pratiqué dans l'insert de pose et d'orientation, permettant le vissage d'une portion filetée correspondante de l'impacteur. On réalise ainsi une structure d'assemblage simple, fiable et peu onéreuse, et qui permet ainsi l'utilisation des impacteurs déjà connus et disponibles.

Selon un premier mode de réalisation avantageux, on peut prévoir que :
- la structure périphérique de réception comporte une gorge annulaire continue ou discontinue,
- l'insert de pose et d'orientation comporte une nervure annulaire continue ou discontinue, engagée élastiquement dans la gorge annulaire.

Selon un second mode de réalisation avantageux, on peut prévoir que :
- la structure périphérique de réception comporte un filetage interne ou externe,
- l'insert de pose et d'orientation comporte un filetage externe ou interne, coopérant avec le filetage de la structure périphérique de réception pourfixer l'insert de pose et d'orientation de façon amovible par vissage sur la structure périphérique de réception.

Les différentes structures périphériques de réception et l'insert de pose et d'orientation sont ainsi faciles et peu onéreux à réaliser, et permettent une tenue suffisante pour que le chirurgien puisse appliquer les efforts nécessaires pour une bonne fixation et une bonne orientation de la prothèse.

On peut avantageusement prévoir que le trou de fixation taraudé est un trou débouchant, apte à coopérer avec un outil de désolidarisation comportant une tige filetée apte à se visser dans le trou de fixation et qui a une extrémité distale conformée pour prendre appui contre lasurface articulaire concave de l'insert articulaire immobile définitif lors du vissage de la tige filetée dans le trou de fixation de l'insert de pose et d'orientation.

En alternative, on peut avantageusement prévoir que :
- l'insert de pose et d'orientation est conformé de façon à ce qu'il reste un espace libre entre l'insert de pose et d'orientation et le fond de la surface articulaire concave de l'insert articulaire immobile définitif une fois l'insert de pose et d'orientation fixé à la structure périphérique de réception de la cupule d'insertion,
- l'insert de pose et d'orientation est en contact de façon étanche selon sa périphérie dans le prolongement annulaire court de la cupule d'insertion,
- le trou de fixation est un trou débouchant, mettant en communication avec l'extérieur l'espace libre entre l'insert de pose et d'orientation et l'insert articulaire immobile définitif, et dimensionné pour y engager l'extrémité d'une seringue de façon étanche.

L'invention propose un procédé de fabrication de cotyle prothétique de hanche.

L'intérêt est à la fois d'assurer une meilleure stérilité de la prothèse, notamment à l'interface entre l'insert articulaire et la cupule d'insertion, tout en permettant l'usage d'un insert articulaire simplement engagé de façon amovible dans la cupule d'insertion.

La stérilisation peut être effectuée par bombardement de rayons gamma. Un tel procédé de stérilisation est en effet compatible avec les matériaux utilisés, à savoir le métal de la cupule d'insertion, et la céramique de l'insert articulaire.

Ce procédé peut être utilisé indépendamment de la présence ou de l'absence des caractéristiques particulières d'épaisseur de l'insert articulaire et de la cupule d'insertion.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue éclatée d'un cotyle avec un insert de pose et d'orientation ;
- la figure 2 est une vue de côté d'une cupule d'insertion ;
- la figure 3 est une vue en coupe des éléments de la figure 1 ;
- la figure 4 est une vue en coupe des éléments de la figure 1 après assemblage ;
- la figure 5 est une vue en coupe partielle d'un cotyle selon l'invention fixé sur un impacteur ;
- la figure 6 est une vue de détail de la figure 5 ;
- les figures 7 et 8 sont des vues en coupe illustrant un premier mode de désolidarisation de l'insert de pose et d'orientation ;
- la figure 9 est une vue en coupe illustrant un second mode de désolidarisation de l'insert de pose et d'orientation ; et
- la figure 10 illustre un cotyle dont l'insert de pose et d'orientation comprend en outre des nervures élastiques de maintien de l'insert articulaire.

### DESCRIPTION DES MODES DE REALISATION PRE-FERES

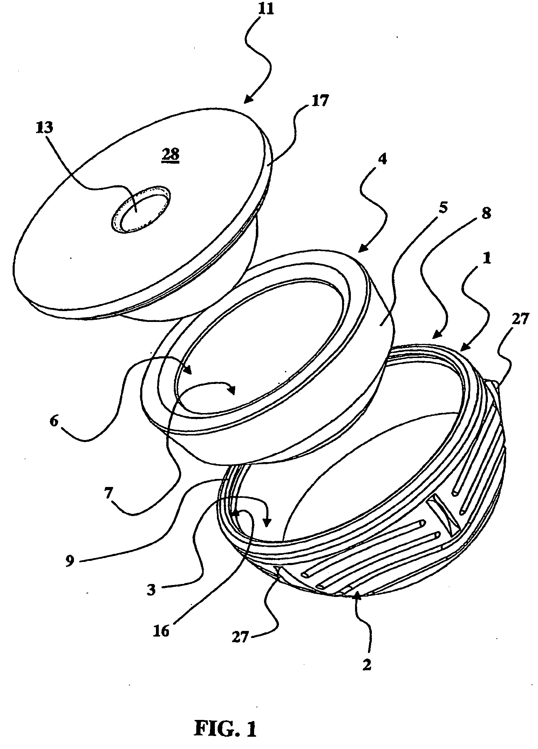

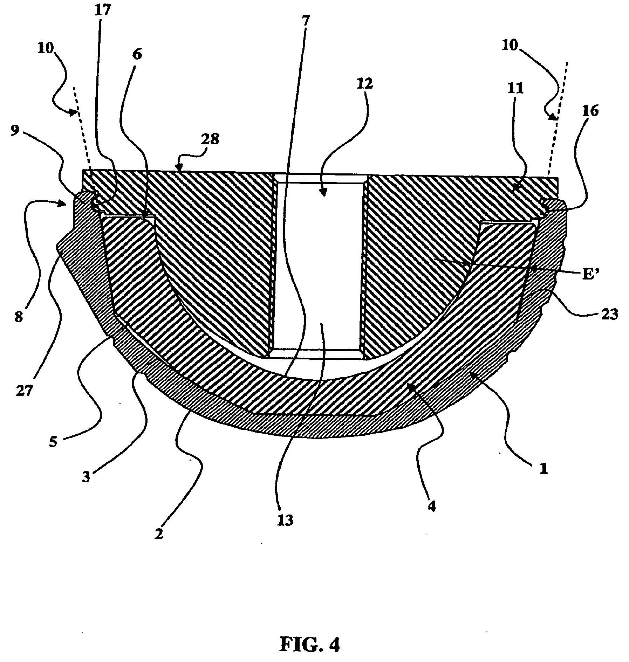

Sur les figures 1, et 4 est représenté un cotyle à simple ou double mobilité. Celui-ci comprend :
- une cupule d'insertion 1, ayant une face extérieure d'ancrage 2 convexe sensiblement hémisphérique conformée pour être ancrée dans une cavité cotyloïdienne du bassin d'un patient, et ayant une face de réception concave 3,
- un insert articulaire 4 immobile définitif en céramique, ayant une face extérieure 5 s'engageant dans la face de réception concave 3 de la cupule d'insertion 1, et ayant une face intérieure réceptrice 6 comportant une surface articulaire concave 7 sensiblement hémisphérique pour permettre l'engagement et le pivotement d'une tête de prothèse fémorale ou d'un insert articulaire mobile (non représentés).

L'insert articulaire 4 définitif est appelé insert articulaire immobile définitif car il est immobile par rapport à la cupule d'insertion 1.

Sur les figures 1, 3 et 4, la cupule d'insertion 1 comporte des ailettes d'ancrage 27 destinées à pénétrer dans l'os de la cavité cotyloïdienne du patient pour assurer un bon ancrage de la cupule d'insertion 1.

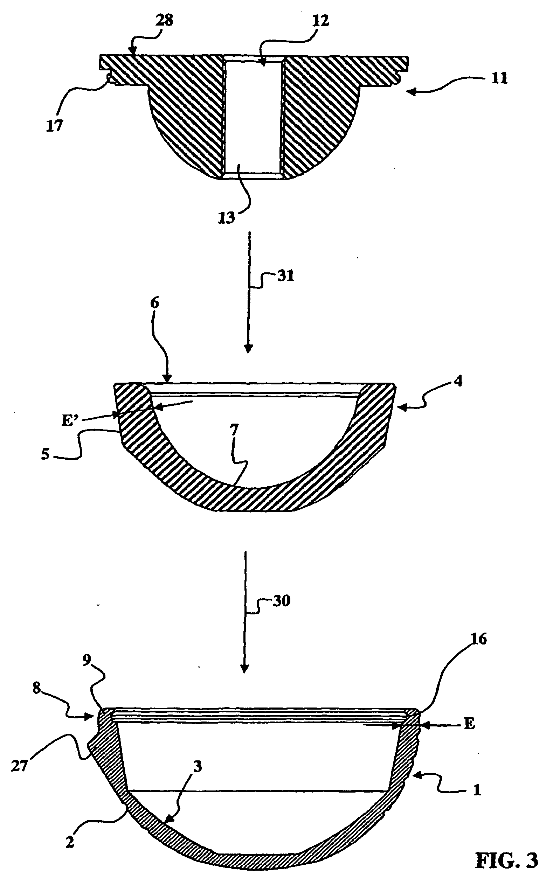

Les figures 1 et 3 sont des vues éclatées respectivement en perspective et en coupe, et la figure 4 est une vue en coupe des éléments des figures 1 et 3 assemblés.

Dans la configuration de la figure 4, le cotyle est conditionné assemblé à l'état stérile avec l'insert articulaire 4 immobile définitif en céramique engagé dans la cupule d'insertion 1. La cupule d'insertion 1 comprend une structure périphérique de réception 8, apte à recevoir des moyens de fixation d'un impacteur de pose de cotyle et conformée de façon que l'impacteur puisse être fixé au cotyle en présence de l'insert articulaire 4 immobile définitif en céramique engagé dans la cupule d'insertion 1.

L'insert articulaire 4 immobile définitif en céramique comporte une faible épaisseur E' pour autoriser l'usage d'une tête fémorale prothétique de grand diamètre.

Pour caractériser l'épaisseur d'un insert articulaire, on considère généralement son épaisseur moyenne selon les différentes directions perpendiculaires à sa surface articulaire concave. Lorsqu'un insert articulaire présente un plat sur son fond, comme dans l'insert articulaire 4 immobile définitif des figures 1, 3 et 4, l'épaisseur de ce fond n'est généralement pas prise en compte pour déterminer son épaisseur moyenne.

L'épaisseurde l'insert articulaire 4 immobile définitif est donc sensiblement égale à l'épaisseur E' représentée sur les figures 3, 4 et 6 à 8.

Pour diminuer efficacement le risque de luxation, on choisit une faible épaisseur E' afin d'augmenter le diamètre de la tête fémorale prothétique (non représentée) destinée à être insérée dans la surface articulaire concave 7 de l'insert articulaire 4 immobile définitif (cas d'un cotyle à simple mobilité du type de celui décrit dans le document EP 1 290 992 A1) ou pour permettre l'utilisation d'un insert articulaire mobile (non représenté) destiné à recevoir la tête fémorale prothétique (cas d'un cotyle à double mobilité du type de celui décrit dans le document WO 2004/069091).

L'épaisseur E' est choisie pour que l'insert articulaire 4 immobile définitif puisse endurer, sans usure ni détérioration prématurées, les contraintes se produisant au cours d'une utilisation normale de prothèse de hanche par un patient.

Par faible épaisseur E', on entend une épaisseur E' inférieure ou égale à 4 mm environ, de préférence inférieure ou égale à 3,5 mm. Pour ce faire, on peut notamment utiliser une céramique telle que celle décrite dans le document EP 1 188 729.

Pour caractériser l'épaisseur d'une cupule d'insertion, on considère généralement son épaisseur au voisinage du bord supérieur de sa face de réception concave. L'épaisseur de la cupule d'insertion 1 est donc sensiblement égale à l'épaisseur E représentée sur les figures 3 et 6 à 9. Cette épaisseur E est l'épaisseur moyenne de la cupule d'insertion 1 au voisinage du bord annulaire 9 de la face concave de réception 3 de la cupule d'insertion 1.

Pour diminuer encore efficacement le risque de luxation pour un cotyle à simple ou double mobilité, on choisit une faible épaisseur E. Par faible épaisseur E, on entend une épaisseur E inférieure ou égale à 4 mm environ, de préférence inférieure ou égale à 3 mm, mais supérieure à 2 mm pour garantir une résistance mécanique suffisante.

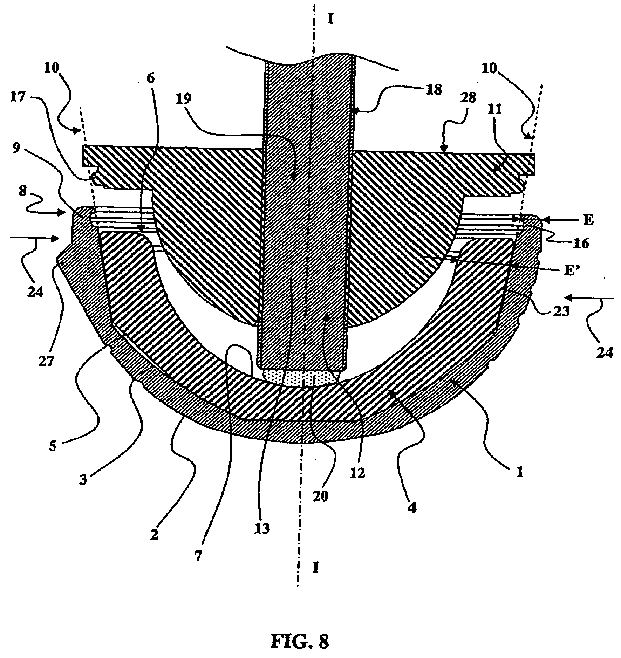

On voit plus particulièrement sur les figures 4 et 8 que la cupule d'insertion 1 comprend une structure périphérique de réception 8 qui est en saillie au-delà de la face réceptrice 6 de l'insert articulaire 4 immobile définitif inséré dans la cupule d'insertion 1.

La structure périphérique de réception 8 constitue un prolongement annulaire court de paroi de cupule d'insertion 1, avec, dans ce mode de réalisation, une face annulaire 10 intérieure de réception (matérialisée par des lignes en pointillés sur les figures 4 et 8) se développant en continuité de la face de réception concave 3 de la cupule d'insertion 1.

Dans les modes de réalisation des figures 1 à 9, pour permettre la fixation d'un impacteur, on prévoit un insert de pose et d'orientation 11, fixé de façon amovible à la structure périphérique de réception 8 de la cupule d'insertion 1. L'insert de pose et d'orientation 11 peut ainsi être solidarisé à la cupule d'insertion 1 en présence de l'insert articulaire 4 immobile définitif en céramique engagé dans la cupule d'insertion 1 (figures 4, 5, 6, 7 et 9), et peut être séparé de la cupule d'insertion 1 (comme il est mieux représenté sur la figure 8) après impaction du cotyle dans la cavité cotyloïdienne du patient.

L'insert de pose et d'orientation 11 comporte une structure d'assemblage 12 sur laquelle peut être fixé un impacteur de façon amovible. L'insert de pose et d'orientation 11, une fois fixé sur la périphérie de la cupule d'insertion 1, répartit sur la cupule d'insertion 1 les efforts appliqués par le chirurgien pour fixer et orienter la prothèse à l'aide d'un impacteur.

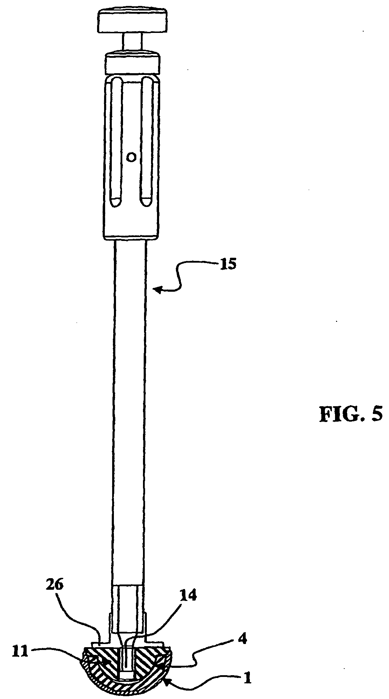

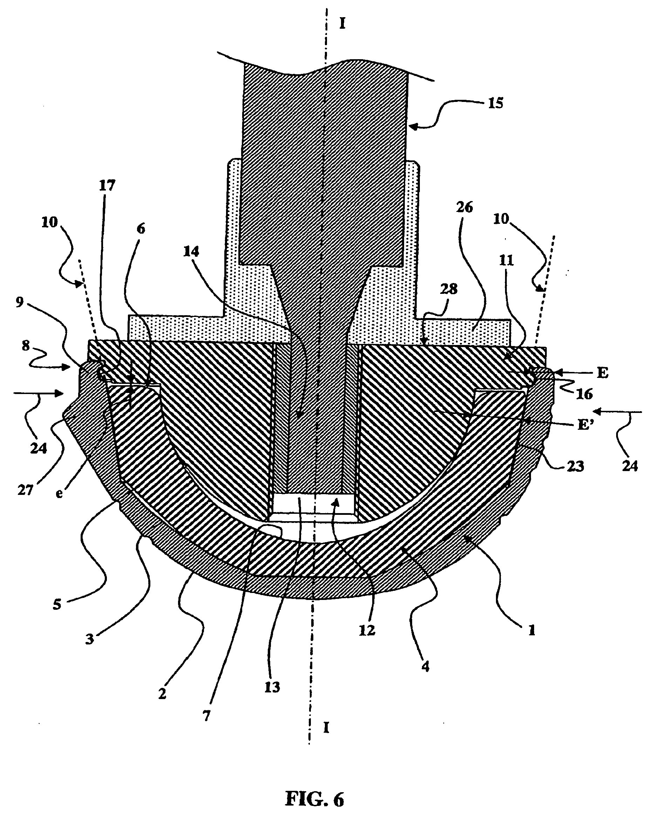

Dans le mode de réalisation représenté sur les figures 1 à 9, la structure d'assemblage 12 comprend un trou de fixation 13 taraudé pratiqué dans l'insert de pose et d'orientation 11. Ce trou de fixation 13 taraudé permet le vissage d'une portion filetée 14 de l'impacteur 15 (figures 5 et 6). Ainsi, pour désolidariser l'impacteur 15 de l'insert de pose et d'orientation 11, il suffit de dévisser la portion filetée 14 de l'impacteur 15 hors du trou defixation 13 taraudé.

Dans le mode de réalisation illustré sur les figures 1 à 9, la structure périphérique de réception 8 comporte une gorge annulaire 16 dans laquelle vient s'engager une nervure annulaire 17 prévue sur l'insert de pose et d'orientation 11. La nervure annulaire 17 vient s'engager élastiquement dans la gorge annulaire 16 lors de l'assemblage de la cupule d'insertion 1, de l'insert articulaire 4 immobile définitif etde l'insert de pose et d'orientation 11, assemblage schématisé sur la figure 3 par les flèches 30 et 31.

Dans le mode de réalisation illustré sur les figures 1 à 9, la gorge annulaire 16 et la nervure annulaire 17 sont continues. Le caractère continu de la gorge annulaire 16 etde la nervure annulaire 17 permet de réaliser une retenue homogène et maximale de l'insert de pose et d'orientation 11 dans la cupule d'insertion 1 selon toute sa périphérie.

Cependant, d'autres formes de nervure annulaire 17 et gorge annulaire 16 peuvent être envisagées. Par exemple, la gorge annulaire 16 et la nervure annulaire 17 peuvent être discontinues.

De même, les dispositions respectives de la gorge annulaire 16 et de la nervure annulaire 17 sur l'insert de pose et d'orientation 11 ou la cupule d'insertion 1 peuvent être différentes de celles représentées sur les figures 1 à 9.

Sur les figures 1 à 9, la gorge annulaire 16 est prévue sur la face annulaire 10 intérieure de réception de la structure périphérique de réception 8 de la cupule d'insertion 1, tandis que la nervure annulaire 17 de l'insert de pose et d'orientation 11 est une nervure annulaire 17 extérieure, engagée élastiquement dans la gorge annulaire 16. L'insert de pose et d'orientation 11 ne déborde pas latéralement au-delà de la périphérie de la cupule d'insertion 1. Il n'y a donc pas de risque de conflit, lors de l'impaction et de l'orientation du cotyle, entre l'insert de pose et d'orientation 11 et la matière osseuse du bassin du patient en périphérie de sa cavité cotyloidienne.

Selon un autre mode de réalisation de l'invention non représenté sur les figures 1 à 9, la structure périphérique de réception 8 et l'insert de pose et d'orientation 11 comportent des filetages coopérant ensemble pour fixer l'insert de pose et d'orientation 11 de façon amovible par vissage sur la structure périphérique de réception 8. Le filetage de la structure périphérique de réception 8 est interne lorsque le filetage de l'insert de pose et d'orientation 11 est externe, et le filetage de la structure périphérique de réception 8 est externe lorsque le filetage de l'insert de pose et d'orientation 11 est un filetage interne.

Lors de son utilisation, le cotyle est conditionné, assemblé à l'état stérile, avec l'insert articulaire 4 immobile définitif en céramique engagé dans la cupule d'insertion 1 (figure 4). Pour poser la cupule d'insertion 1 dans la cavité cotyloïdienne, le chirurgien utilise un impacteur 15 avec un tronçon d'extrémité 14 fileté qui vient se fixer dans le trou de fixation 13 taraudé pratiqué dans l'insert de pose et d'orientation 11 (figures 5 et 6).

L'impacteur 15 comporte un flasque 26 en appui contre la face supérieure 28 de l'insert de pose et d'orientation 11. Le flasque 26 permet de répartir les efforts d'impaction et d'orientation sur l'insert de pose et d'orientation 11.

L'impacteur 15 permet au chirurgien d'appliquer une force d'enfoncement de la cupule d'insertion 1 dans la cavité cotyloïdienne de l'os, permet d'appliquer des couples de rotation pour régler l'orientation de la cupule d'insertion 1, et permet de maintenir la cupule d'insertion 1 en position fixe pendant une durée suffisante notamment pour la prise d'un ciment entre la surface externe de la cupule d'insertion 1 et la cavité cotyloïdienne de l'os.

Lors de ces opérations, la cupule d'insertion 1 subit des efforts importants qui, du fait de la faible épaisseur E (figure 6), pourraient déformer la cupule d'insertion 1 seule au contact de l'os du bassin du patient. Cette déformation de la cupule d'insertion 1 est empêchée, lors de l'impaction de la cupule d'insertion 1, par la présence de l'insert articulaire 4 immobile définitif engagé dans la cupule d'insertion 1.

Ainsi, si l'os du bassin du patient a tendance à déformer la cupule d'insertion 1 du fait de sa faible épaisseur E, l'insert articulaire 4 immobile définitif en céramique est suffisamment rigide et solide pour contrer toute déformation. L'insert articulaire 4 immobile définitif en céramique rigidifie la cupule d'insertion 1.

La cupule d'insertion 1, l'insert articulaire 4 immobile définitif et l'insert de pose et d'orientation 11 peuvent avantageusement être conditionnés ensemble à l'état stérile. Le chirurgien peut alors impacter en une seule opération la cupule d'insertion 1 avec son insert articulaire 4 immobile définitif. Le chirurgien gagne ainsi un temps opératoire précieux, ce qui contribue à réduire les risques opératoires liés à une possible infection.

Une fois que la cupule d'insertion 1 munie de son insert articulaire 4 immobile définitif a été impactée, le chirurgien retire l'insert de pose et d'orientation 11 hors de la structure périphérique de réception 8 de la cupule d'insertion 1 et vient loger la tête fémorale prothétique choisie dans la surface articulaire concave 7 de l'insert articulaire 4 immobile définitif.

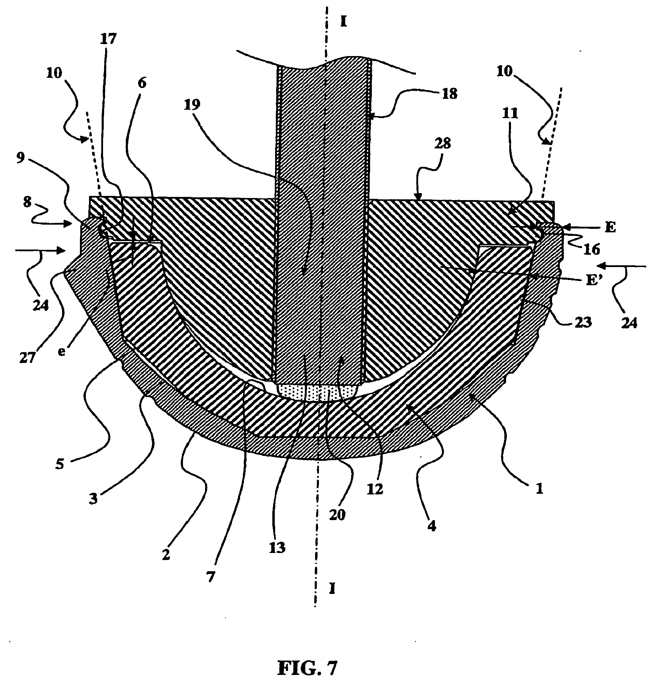

L'étape de désolidarisation de l'insert de pose et d'orientation 11 et de la cupule d'insertion 1 est représentée plus particulièrement sur les figures 7 à 9.

Un premier mode de désolidarisation de l'insert de pose et d'orientation 11 est illustré sur les figures 7 et 8.

Sur ces figures 7 et 8, le caractère débouchant du trou de fixation 13 taraudé permet d'engager un outil de désolidarisation 18 comportant une tige filetée 19 apte à se visser dans le trou de fixation 13. L'outil de désolidarisation 18 comporte une extrémité distale 20 conformée pourprendre appui contre la surface articulaire concave 7 de l'insert articulaire 4 immobile définitif lors du vissage de la tige filetée 19 dans le trou de fixation 13 de l'insert de pose et d'orientation 11.

Le fait de visser la tige filetée 19 dans le trou de fixation 13 (en tournant dans un sens ou dans l'autre en fonction de la configuration des filets utilisés) permet d'écarter l'insert de pose et d'orientation 11 de la cupule d'insertion 1 selon la direction axiale I-I jusqu'à sa désolidarisation complète (figure 8) où la nervure annulaire 17 de l'insert de pose et d'orientation 11 a échappé élastiquement à la gorge annulaire 16.

L'extrémité distale 20 de l'outil de désolidarisation 18 est adaptée pour prendre appui contre la surface articulaire concave 7 de l'insert articulaire 4 immobile définitif sans pour autant dégrader celle-ci.

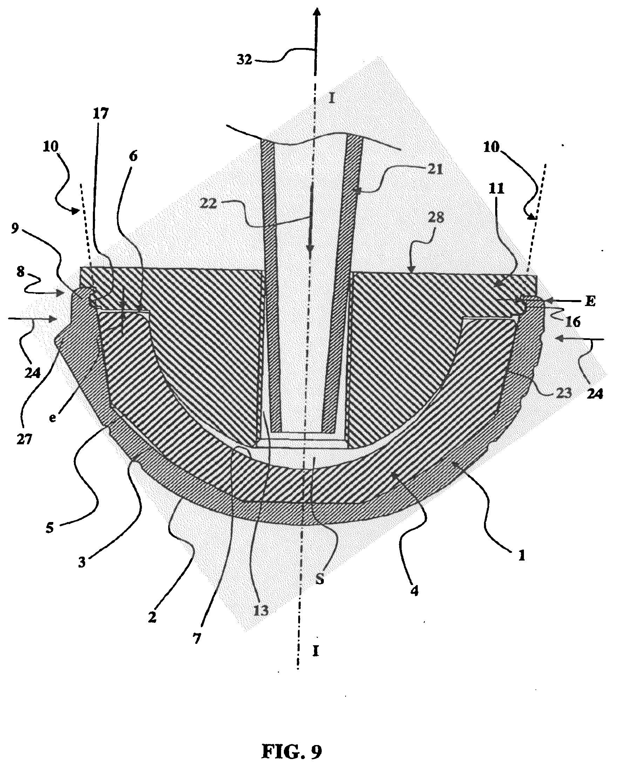

Un second mode de désolidarisation de l'insert de pose et d'orientation 11 est illustré sur la figure 9.

Sur cette figure 9, l'insert de pose et d'orientation 11 est conformé de façon à ce qu'il reste un espace libre S entre l'insert de pose et d'orientation 11 et le fond de la surface articulaire concave 7 de l'insert articulaire 4 immobile définitif une fois l'insert de pose et d'orientation 11 fixé à la structure périphérique de réception 8 de la cupule d'insertion 1. L'insert de pose et d'orientation 11 est en contact de façon étanche selon sa périphérie dans le prolongement annulaire court 9 de la cupule d'insertion 1.

Le caractère débouchant du trou de fixation 13 permet de mettre en communication avec l'extérieur l'espace libre S ménagé entre l'insert de pose et d'orientation 11 et l'insert articulaire 4 immobile définitif.

Le trou de fixation 13 est dimensionné pour permettre l'engagement de l'extrémité d'une conduite 21 d'amenée de fluide sous pression.

La conduite 21 permet d'amener un fluide depuis l'extérieur dans l'espace libre S comme illustré par la flèche 22. Le fluide va alors remplir l'espace libre S, et la pression de fluide dans la conduite 21 et dans l'espace libre S va provoquer une poussée d'axe I-I sur l'insert de pose et d'orientation 11 dans le sens défini par la flèche 32. Avec une pression de fluide suffisante et une surface d'insert de pose et d'orientation 11 suffisante sur laquelle peut s'appliquer cette pression, on retire rapidement et facilement l'insert de pose et d'orientation 11 de la cupule d'insertion 1 en faisant sortir la nervure annulaire 17 hors de la gorge annulaire 16.

En pratique, on a obtenu de bons résultats par l'utilisation d'une seringue. La conduite d'amenée de fluide 21 peut donc être l'extrémité d'une seringue. L'utilisation d'une seringue s'avère particulièrement intéressante dans le cadre médical. Il s'agit en effet d'un objet médical courant, qui peut être stérilisé et qui est facilement utilisable dans un bloc opératoire. En alternative, la conduite d'amenée de fluide 21 peut être une partie tubulaire de l'impacteur lui-même.

Le fluide utilisé pour la désolidarisation de la cupule d'insertion 1 et de l'insert de pose et d'orientation 11 peut être de l'eau ou du sérum physiologique qui sont des fluides utilisables et très répandus dans les blocs opératoires.

En pratique, on a obtenu de bons résultats en utilisant un insert de pose et d'orientation 11 en polyéthylène. Le polyéthylène est en effet un matériau admissible en bloc opératoire, de faible coût et qui peut être facilement stérilisé.

Sur la figure 6, on voit que l'insert de pose et d'orientation 11 est fixé sur la structure périphérique de réception 8 de la cupule d'insertion 1 à faible écart e de la face intérieure réceptrice 6 de l'insert articulaire 4 immobile définitif en céramique. Un tel écart e permet un engagement fiable et sûr de la nervure annulaire 17 dans la gorge annulaire 16 selon toute sa périphérie. En outre, lors de l'impaction du cotyle dans la cavité cotyloïdienne du bassin d'un patient, l'insert de pose et d'orientation 11 se déformera très légèrement sous les efforts d'impaction axiaux d'axe I-I pour venir à un écart encore plus faible de ou en appui sur la face intérieure réceptrice 6 de l'insert articulaire 4 immobile définitif en céramique ou sur son bord périphérique.

Dans le cas où l'insert de pose et d'orientation 11 en polyéthylène vient en appui sur la face intérieure réceptrice 6 de l'insert articulaire 4 immobile définitif en céramique ou sur son bord périphérique, l'insert articulaire 4 immobile définitif permet la transmission des efforts d'impaction et d'orientation du cotyle. L'insert articulaire 4 immobile définitif est en outre maintenu plaqué contre la face de réception concave 3 de la cupule d'insertion 1, ce qui garantit que l'insert articulaire 4 immobile définitif participe à la rigidité de la cupule d'insertion 1 et empêche toute déformation de celle-ci.

Dans le cas où il subsiste, lors de l'impaction du cotyle, un écart e important entre l'insert de pose et d'orientation 11 et la face intérieure réceptrice 6 de l'insert articulaire 4 immobile définitif en céramique, on peut prévoir, comme représenté sur les figures 4 et 6 à 9, un emmanchement conique 23 entre l'insert articulaire 4 immobile définitif et la cupule d'insertion 1. On est ainsi sûr, malgré la présence de l'écart e et des chocs provoqués par l'impaction, que l'insert articulaire 4 immobile définitif participe à la rigidité de la cupule d'insertion 1 et empêche toute déformation de celle-ci notamment contre les déformations pouvant se produire du fait des efforts radiaux, illustrés par les flèches 24, induits par la matière osseuse du bassin du patient.

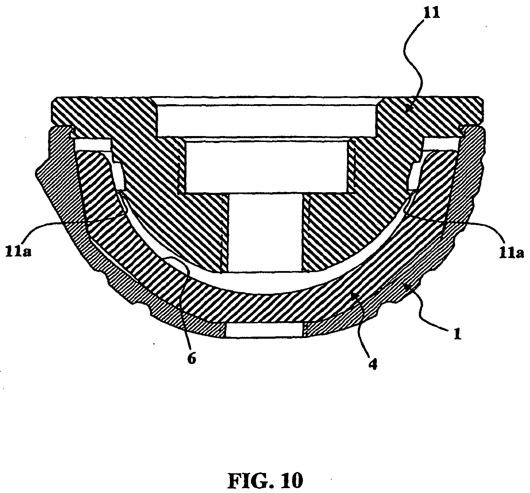

Dans le mode de réalisation illustré sur la figure 10, l'insert de pose et d'orientation 11 comprend une nervure élastique 11 a en saillie qui vient en appui élastique contre l'insert articulaire 4 en céramique afin de le maintenir plaqué dans la cupule d'insertion 1. Dans la réalisation illustrée, la nervure 11 a est en forme de lèvre annulaire, et vient en appui contre la face intérieure réceptrice 6. En alternative, on peut prévoir une nervure venant en appui sur le bord frontal périphérique de l'insert articulaire 4 en céramique, ou on peut prévoir plusieurs nervures ou autres formes élastiques venant en appui simultané sur l'insert articulaire 4.

On comprend que, contrairement aux cotyles à simple mobilité généralement connus, la présence de l'insert articulaire 4 immobile définitif en céramique engagé dans la cupule d'insertion 1 interdit l'utilisation de vis de fixation pour fixer la cupule d'insertion 1 dans la cavité cotyloïdienne du bassin du patient en passant à travers des trous traversant la cupule d'insertion depuis sa face de réception concave vers sa face extérieure d'ancrage 2 convexe.

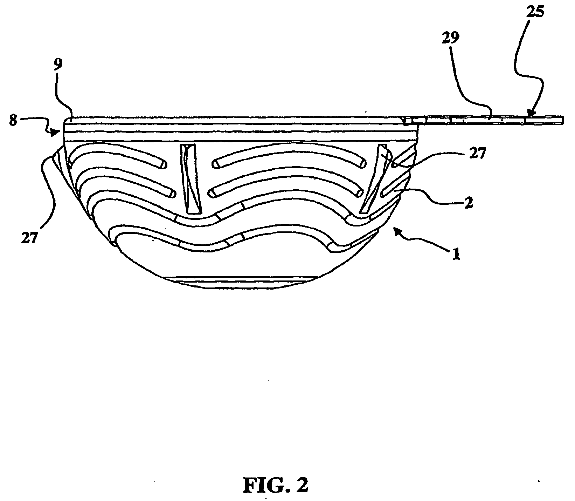

Dans ce cas, il peut être utile de prévoir, au voisinage du bord supérieur de la face extérieure d'ancrage 2 convexe de la cupule d'insertion 1, une patte de fixation externe 25 illustrée sur la figure 2. Cette patte de fixation externe 25 comporte une lumière 29 que traverse une vis (non représentée) pour venir en prise dans l'os du bassin du patient.

Bien que seuls quelques modes de fixation de l'insert de pose et d'orientation 11 sur la cupule d'insertion 1 aient été explicitement décrits, on comprendra que tout autre mode de fixation alternatif est compris dans le champ de protection de l'invention. Par exemple, on pourra réaliser la fixation de l'insert de pose et d'orientation 11 sur la structure périphérique de réception 8 de la cupule d'insertion 1 par une insertion en force de l'insert de pose et d'orientation 11.

La cupule d'insertion 1 peut être métallique, en titane ou en un alliage d'acier au chrome et au cobalt. La cupule d'insertion 1 peut également être en PEEK (polyétheréthercétone) ou en tout autre matériau adapté. La face convexe externe 2 de la cupule d'insertion 1 peut avantageusement être recouverte d'une couche d'hydroxyapatite (HAP).

En pratique, on a obtenu de bonnes capacités anti-luxation et une bonne résistance à la déformation lors de l'impaction avec un insert articulaire 4 immobile définitif en céramique d'épaisseur E' inférieure ou égale à 3,5 mm engagé dans une cupule d'insertion 1 métallique d'épaisseur E inférieure ou égale à 4 mm.

La présente invention n'est pas limitée aux modes de réalisation qui ont été explicitement décrits, mais elle en inclut les diverses variantes et généralisations contenues dans le domaine des revendications ci-après.

## Revendications

1. Procédé de fabrication de cotyle prothétique de hanche, comprenant les étapes de :
a) prévoir une cupule d'insertion (1) métallique ayant une face de réception concave (3) et une structure périphérique de réception (8),
b) prévoir un insert articulaire (4) en céramique destiné à être immobile et définitif ayant une face extérieure (5) et une face intérieure réceptrice (6) comportant une surface articulaire concave (7),
c) engager la face extérieure (5) de l'insert articulaire (4) dans la face de réception concave (3) de la cupule d'insertion (1),
c1) fixer de façon réversible à la structure périphérique de réception (8) de la cupule d'insertion (1) un insert de pose et d'orientation (11) comportant une structure d'assemblage (12) sur laquelle un impacteur (15) peut être fixé de façon amovible,
d) stériliser l'ensemble ainsi formé dans une enveloppe de protection microbienne.

2. Procédé selon la revendication 1, **caractérisé en ce que** l'étape d) de stérilisation est réalisée par bombardement de rayons gamma.

3. Procédé selon l'une des revendications 1 ou 2, **caractérisé en ce que** l'insert de pose et d'orientation (11) est en polyéthylène.

4. Procédé selon l'une quelconque des revendications 1 à 3, **caractérisé en ce que** la structure d'assemblage (12) comprend un trou de fixation (13) taraudé pratiqué dans l'insert de pose et d'orientation (11), permettant le vissage d'une portion filetée (14) correspondante de l'impacteur (15).

5. Procédé selon l'une quelconque des revendications 1 à 4, **caractérisé en ce que** l'insert de pose et d'orientation (11) fixé sur la cupule d'insertion (1) est en appui sur la face intérieure réceptrice (6) de l'insert articulaire (4) immobile définitif en céramique.

6. Procédé selon la revendication 1 ou selon l'une des revendications 3 ou 4 dans leur rattachement à la revendication 1, **caractérisé en ce que** :
- l'insert de pose et d'orientation (11) fixé sur la cupule d'insertion (1) est à faible écart (e) de la face intérieure réceptrice (6) de l'insert articulaire (4) immobile définitif en céramique,
- l'étape d) de stérilisation est réalisée par bombardement de rayons gamma.

7. Procédé selon l'une quelconque des revendications 1 à 6, **caractérisé en ce que** l'insert de pose et d'orientation (11), fixé sur la cupule d'insertion (1), comporte une ou plusieurs nervures élastiques (11a) en saillie venant en appui sur l'insert articulaire (4) immobile définitif en céramique pour le maintenir dans la cupule d'insertion (1).

8. Procédé selon l'une quelconque des revendications 1 à 7, **caractérisé en ce que** la cupule d'insertion (1) a, au voisinage d'un bord annulaire (9), une épaisseur (E) inférieure ou égale à 4 mm environ et supérieure à 2 mm.

9. Procédé selon l'une quelconque des revendications 1 à 8, **caractérisé en ce que** l'insert articulaire (4) immobile définitif en céramique a une épaisseur (E') inférieure ou égale à 4 mm environ.

10. Procédé selon l'une quelconque des revendications 1 à 9, **caractérisé en ce que** la structure périphérique de réception (8) est annulaire.

11. Procédé selon l'une quelconque des revendications 1 à 10, **caractérisé en ce que** :
- la structure périphérique de réception (8) comporte une gorge annulaire (16) continue ou discontinue,
- l'insert de pose et d'orientation (11) comporte une nervure annulaire (17) continue ou discontinue, engagée élastiquement dans la gorge annulaire (16).

12. Procédé selon la revendication 11, **caractérisé en ce que** :
- la gorge annulaire (16) est continue,
- la nervure annulaire (17) de l'insert de pose et d'orientation (11) est une nervure annulaire (17) extérieure continue, engagée élastiquement dans la gorge annulaire (16).

13. Procédé selon l'une quelconque des revendications 1 à 10, **caractérisé en ce que** :
- la structure périphérique de réception (8) comporte un filetage interne ou externe,
- l'insert de pose et d'orientation (11) comporte un filetage externe ou interne, coopérant avec le filetage de la structure périphérique de réception (8) pour fixer l'insert de pose et d'orientation (11) de façon amovible par vissage sur la structure périphérique de réception (8).

14. Procédé selon la revendication 4, **caractérisé en ce que** le trou de fixation (13) taraudé est un trou débouchant, apte à coopérer avec un outil de désolidarisation (18) comportant une tige filetée (19) apte à se visser dans le trou de fixation (13) et qui a une extrémité distale (20) conformée pour prendre appui contre la surface articulaire concave (7) de l'insert articulaire (4) immobile définitif lors du vissage de la tige filetée (19) dans le trou de fixation (13) de l'insert de pose et d'orientation (11).

15. Procédé selon la revendication 4, **caractérisé en ce que** :
- l'insert de pose et d'orientation (11) est conformé de façon à ce qu'il reste un espace libre (S) entre l'insert de pose et d'orientation (11) et le fond de la surface articulaire concave (7) de l'insert articulaire (4) immobile définitif une fois l'insert de pose et d'orientation (11) fixé à la structure périphérique de réception (8) de la cupule d'insertion (1),
- l'insert de pose et d'orientation (11) est en contact de façon étanche selon sa périphérie dans la structure périphérique de réception (8) de la cupule d'insertion (1),
- le trou de fixation (13) est un trou débouchant, mettant en communication avec l'extérieur l'espace libre (S) entre l'insert de pose et d'orientation (11) et l'insert articulaire (4) immobile définitif, et dimensionné pour y engager l'extrémité d'une seringue de façon étanche.

## Patentansprüche

1. Verfahren zur Herstellung einer prothetischen Hüftgelenkpfanne mit folgenden Schritten:
a) Bereitstellen eines metallischen Pfanneneinsatzes (1), der eine konkave Aufnahmefläche (3) und eine periphere Aufnahmestruktur (8) aufweist,
b) Bereitstellen eines Gelenkeinsatzes (4) aus Keramik, der unbeweglich und endgültig sein wird, der eine äußere Fläche (5) hat, und eine innere Aufnahmefläche (6) hat, die eine konkave Gelenkfläche (7) hat,
c) Einsetzen der äußeren Fläche (5) des Gelenkeinsatzes (4) in die konkave Aufnahmefläche (3) des Pfanneneinsatzes (1),
c1) Befestigen eines Positionierungs- und Orientierungs-einsatzes (11) an der peripheren Aufnahmestruktur (8) des Pfanneneinsatzes (1) in reversibler Weise, der eine Montagestruktur (12) trägt, an der ein Montageschaft (15) in abnehmbarer Weise fixiert werden kann,
d) Sterilisieren der so geformten Gesamtheit in einer mikrobiologischen Schutzhülle.

2. Verfahren nach Anspruch 1, **dadurch gekennzeichnet, daß** der Schritt d) der Sterilisation durch Bestrahlung mit Gamma-Strahlen durchgeführt wird.

3. Verfahren nach einem der Ansprüche 1 oder 2, **dadurch gekennzeichnet, daß** der Positionierungs- und Orientierungseinsatz (11) aus Polyethylen ist.

4. Verfahren nach irgendeinem der Ansprüche 1 bis 3, **dadurch gekennzeichnet, daß** die Montagestruktur (12) ein Befestigungsloch (13) mit Gewinde aufweist, das in dem Positionierungs- und Orientierungseinsatz (11) angebracht ist und das Einschrauben eines Gewindeteiles (14) entsprechend dem Montageschaft (15) erlaubt.

5. Verfahren nach irgendeinem der Ansprüche 1 bis 4, **dadurch gekennzeichnet, daß** der Positionierungs- und Orientierungseinsatz (11), der an dem Pfanneneinsatz (1) befestigt ist, im Anschlag zur inneren Aufnahmefläche (6) des endgültig unbeweglichen Gelenkeinsatzes (4) aus Keramik ist.

6. Verfahren nach Anspruch 1 oder nach einem der Ansprüche 3 oder 4 nach ihrer Verbindung mit dem Anspruch 1, **dadurch gekennzeichnet, daß**:
- der Positionierungs- und Orientierungseinsatz (11), der an dem Pfanneneinsatz (1) befestig ist, in geringem Abstand (e) zur inneren Aufnahmefläche (6) des endgültig unbeweglichen Gelenkeinsatzes (4) aus Keramik ist,
- der Schritt d) der Sterilisation durch Bestrahlung mit Gamma-Strahlen durchgeführt wird.

7. Verfahren nach irgendeinem der Ansprüche 1 bis 6, **dadurch gekennzeichnet, daß** dem Positionierungs- und Orientierungseinsatz (11), der an dem Pfanneneinsatz (1) befestigt ist, eine oder mehrere elastische überstehende Rippen (11a) hat, die an dem endgültig unbeweglichen Gelenkeinsatz (4) aus Keramik in Anschlag kommen, um ihn in dem Pfanneneinsatz (1) zu halten.

8. Verfahren nach irgendeinem der Ansprüche 1 bis 7, **dadurch gekennzeichnet, daß** der Pfanneneinsatz (1) in Nachbarschaft zu ihrem ringförmigen Rand (9) eine Dicke (E) hat, die kleiner oder gleich ungefähr 4 mm ist und größer als 2 mm.

9. Verfahren nach irgendeinem der Ansprüche 1 bis 8, **dadurch gekennzeichnet, daß** der endgültig unbewegliche Gelenkeinsatz (4) aus Keramik eine Dicke (E') von kleiner oder gleich ungefähr 4 mm hat.

10. Verfahren nach irgendeinem der Ansprüche 1 bis 9, **dadurch gekennzeichnet, daß** die periphere Aufnahmestruktur (8) ringförmig ist.

11. Verfahren nach irgendeinem der Ansprüche 1 bis 10, **dadurch gekennzeichnet, daß**:
- die periphere Aufnahmestruktur (8) eine ringförmige kontinuierliche oder diskontinuierliche Hohlkehle (16) hat,
- der Positionierungs- und Orientierungseinsatz (11) einen ringförmigen, kontinuierlichen oder diskontinuierlichen Vorsprung (17) hat, der elastisch in die ringförmige Hohlkehle (16) eingreift.

12. Verfahren nach Anspruch 11, **dadurch gekennzeichnet, daß**:
- die ringförmige Hohlkehle (16) kontinuierlich ist,
- der ringförmige Vorsprung (17) des Positionierungs- und Orientierungseinsatzes (11) ein äußerer kontinuierlicher Vorsprung (17) ist, der elastisch in die ringförmige Hohlkehle (16) eingreift.

13. Verfahren nach irgendeinem der Ansprüche 1 bis 10, **dadurch gekennzeichnet, daß**:
- die periphere Aufnahmestruktur (8) ein internes oder externes Gewinde aufweist,
- der Positionierungs- und Orientierungseinsatz (11) eine äußeres oder inneres Gewinde aufweist, das mit dem Gewinde der periphere Aufnahmestruktur (8) zusammen arbeitet, um den Positionierungs- und Orientierungseinsatz (11) in abnehmbarer Weise mittels Einschrauben an der peripheren Aufnahmestruktur (8) zu befestigen.

14. Verfahren nach der Anspruch 4, **dadurch gekennzeichnet, daß** das Befestigungsloch (13), das mit einem Gewinde versehen ist, ein Durchgangsloch ist, das ausgebildet ist, um mit einem Ausbauwerkzeug (18) zusammen zu arbeiten, das einen Gewindeschaft (19) aufweist, der ausgebildet ist, um sich in das Befestigungsloch (13) einzuschrauben und der ein distales Ende (20) aufweist, das ausgebildet ist, um in Anschlag gegen die konkave Gelenkfläche (7) des endgültig unbeweglichen Gelenkeinsatzes (4) in Anschlag zu kommen, beim Einschrauben des Gewindeschaftes (19) in das Befestigungsloch (13) des Positionierungs- und Orientierungseinsatzes (11).

15. Verfahren nach Anspruch 4, **dadurch gekennzeichnet, daß**:
- der Positionierungs- und Orientierungseinsatz (11) derart ausgebildet ist, daß er einen freien Raum (S) zwischen dem Positionierungs- und Orientierungseinsatz (11) und dem Boden der konkaven Gelenkfläche (7) des endgültig unbeweglichen Gelenkeinsatzes (4) läßt, wenn der Positionierungs- und Orientierungseinsatz (11) an der peripheren Aufnahmestruktur (8) des Pfanneneinsatzes (1) befestigt ist,
- der Positionierungs- und Orientierungseinsatz (11) mit seinem Umfang in dichtem Kontakt in der peripheren Aufnahmestruktur (8) des Pfanneneinsatzes (1) ist,
- das Befestigungsloch (13) ein Durchgangsloch ist, das eine Verbindung mit dem äußeren freien Raum (S) zwischen dem Positionierungs- und Orientierungseinsatz (11) und dem endgültig unbeweglichen Gelenkeinsatz (4) läßt und dimensioniert ist, um dort das Ende einer Spritze in dichter Weise eingreifen zu lassen.

## Claims

1. Method of producing a prosthetic hip cotyle, comprising the steps of:
a) providing a metal insertion shell (1) having a concave receiving face (3) and an peripheral receiving structure (8),
b) providing a ceramic joint insert (4) intended to be fixed and final having an exterior face (5) and an interior receiving face (6) comprising a concave joint surface (7),
c) engaging the exterior face (5) of the joint insert (4) in the concave receiving face (3) of the insertion shell (1),
c1) removably fixing to the peripheral receiving structure (8) of the insertion shell (1) a placement and orientation insert (11) having an assembly structure (12) to which an impacter (15) can be removably fixed,
d) sterilizing the combination thus formed in a microbial protection envelope.

2. Method according to claim 1, **characterized in that** the sterilization step d) is effected by bombardment with gamma rays.

3. Method according to either of claims 1 or 2, **characterized in that** the placement and orientation insert (11) is made of polyethylene.

4. Method according to any one of claims 1 to 3, **characterized in that** the assembly structure (12) comprises a threaded fixing hole (13) formed in the placement and orientation insert (11), enabling the screwing in of a corresponding threaded portion (14) of the impacter (15).

5. Method according to any one of claims 1 to 4, **characterized in that** the placement and orientation insert (11) fixed to the insertion shell (1) bears on the interior receiving face (6) of the ceramic fixed final joint insert (4).

6. Method according to claim 1 or according to either of claims 3 and 4 when depending on claim 1, **characterized in that**:
- the placement and orientation insert (11), fixed to the insertion shell (1), is at a small distance (e) from the interior receiving face (6) of the ceramic fixed final joint insert (4),
- the sterilization step d) is effected by bombardment with gamma rays.

7. Method according to any one of claims 1 to 6, **characterized in that** the placement and orientation insert (11), fixed to the insertion shell (1), includes one or more projecting elastic ribs (11a) adapted to come to bear on the ceramic fixed final joint insert (4) to retain it in the insertion shell (1).

8. Method according to any one of claims 1 to 7, **characterized in that** the insertion shell (1) has, in the vicinity of an annular edge (9), a thickness (E) less than or equal to approximately 4 mm and greater than 2 mm.

9. Method according to any one of claims 1 to 8, **characterized in that** the ceramic fixed final joint insert (4) has a thickness (E') less than or equal to approximately 4 mm.

10. Method according to any one of claims 1 to 9, **characterized in that** the peripheral receiving structure (8) is annular.

11. Method according to any one of claims 1 to 10, **characterized in that**:
- the peripheral receiving structure (8) includes a continuous or discontinuous annular groove (16),
- the placement and orientation insert (11) includes a continuous or discontinuous annular rib (17), elastically engaged in the annular groove (16).

12. Method according to claim 11, **characterized in that**:
- the annular groove (16) is continuous,
- the annular rib (17) of the placement and orientation insert (11) is a continuous exterior annular rib (17), elastically engaged in the annular groove (16).

13. Method according to any one of claims 1 to 10, **characterized in that**:
- the peripheral receiving structure (8) includes an internal or external thread,
- the placement and orientation insert (11) includes an external or internal thread, cooperating with the thread of the peripheral receiving structure (8) to fix the placement and orientation insert (11) removably by screwing it to the peripheral receiving structure (8).

14. Method according to claim 4, **characterized in that** the threaded fixing hole (13) is an open hole, adapted to cooperate with a detachment tool (18) including a threaded rod (19) adapted to be screwed into the fixing hole (13) and that has a distal end (20) conformed to bear against the concave joint surface (7) of the fixed final joint insert (4) during screwing of the threaded rod (19) into the fixing hole (13) of the placement and orientation insert (11).

15. Method according to claim 4, **characterized in that**:
- the placement and orientation insert (11) is conformed so that there remains a free space (S) between the placement and orientation insert (11) and the bottom of the concave joint surface (7) of the fixed final joint insert (4) once the placement and orientation insert (11) is fixed to the peripheral receiving structure (8) of the insertion shell (1),
- the placement and orientation insert (11) is in sealed contact at its periphery in the peripheral receiving structure (8) of the insertion shell (1),
- the fixing hole (13) is an open hole, providing communication between the exterior and the free space (S) between the placement and orientation insert (11) and the fixed final joint insert (4), and sized to engage therein in sealed manner the end of a syringe.
